(19)

**Europäisches Patentamt**
**European Patent Office**
**Office européen des brevets**

(11) **EP 4 491 112 A1**

(12) **DEMANDE DE BREVET EUROPEEN**

(43) Date de publication:
**15.01.2025 Bulletin 2025/03**

(21) Numéro de dépôt: **23306220.7**

(22) Date de dépôt: **13.07.2023**

(51) Classification Internationale des Brevets (IPC):
**A61B 5/11** (2006.01)  **A61B 5/00** (2006.01)

(52) Classification Coopérative des Brevets (CPC):
**A61B 5/4082; A61B 5/1101; A61B 5/4839;**
A61B 5/7275

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(71) Demandeur: **Diampark**
**92370 Chaville (FR)**

(72) Inventeur: **DALILI, Djamchid**
**92370 CHAVILLE (FR)**

(74) Mandataire: **Icosa**
**83 avenue Denfert-Rochereau**
**75014 Paris (FR)**

(54) **PROCEDE ET DISPOSITIF POUR DETERMINER UNE DOSE RECOMMANDEE DE MEDICAMENT POUR LE TRAITEMENT DE LA MALADIE DE PARKINSON**

(57) La présente invention concerne un procédé mis en oeuvre par ordinateur pour déterminer, à un instant courant t, des informations concernant une dose de médicament pour le traitement de la maladie de Parkinson.

FIG. 3

**Description**

**DOMAINE DE L'INVENTION**

La présente invention concerne le traitement de la maladie de Parkinson. La présente invention porte sur un procédé mis en oeuvre par ordinateur pour déterminer, à un instant courant t, des informations concernant une dose de médicament pour le traitement de la maladie de Parkinson et un dispositif associé.

**ÉTAT DE LA TECHNIQUE**

La maladie de Parkinson est une pathologie chronique, qui s'installe progressivement et affecte le système nerveux central en provoquant la dégénérescence de certains neurones du système nerveux central. La conséquence principale de cette disparition neuronale est la diminution de la production de dopamine, une molécule qui permet aux neurones de communiquer entre eux, dans une région essentielle au contrôle des mouvements. Les symptômes de la maladie de Parkinson sont caractéristiques, même s'ils peuvent être variables d'un patient à l'autre : une difficulté à initier un mouvement (akinésie) ; un ralentissement des gestes ; une écriture de plus en plus petite et difficile ; une rigidité des membres, bras ou jambe (hypertonie) ; des tremblements caractéristiques qui apparaissent au repos, lorsque les muscles sont relâchés.

Il n'existe pas, à ce jour, de traitement curatif contre la maladie de Parkinson. Seules existent des thérapies qui permettent de limiter l'avancée de la maladie. Le traitement par la lévodopa (ou L-dopa), molécule précurseur de la dopamine, est le plus répandu. La lévodopa est transformée en dopamine dans le cerveau où l'apport de cette dopamine de synthèse compense la carence en dopamine naturelle. La lévodopa agit sur l'akinésie et l'hypertonie mais également sur les tremblements. Pour que son effet soit plus prolongé, elle est généralement administrée en combinaison avec une ou plusieurs substances, telles que le bensérazide, la carbidopa ou l'entacapone, qui inhibent sa dégradation par l'organisme jusqu'à ce qu'elle atteigne le cerveau.

Le suivi de la réaction d'un patient parkinsonien à un traitement par la lévodopa est important afin d'estimer l'efficacité du traitement et d'ajuster la posologie (dose et fréquence d'administration).

Il pourrait s'avérer avantageux de tirer parti de données du quotidien de patients atteints de la maladie de Parkinson et prenant un traitement incluant notamment la lévodopa, pour le suivi des effets de ce traitement. En effet, la « vraie vie » (en anglais « real-world evidence », ou RWE) permet d'obtenir des informations utiles complémentaires aux données d'essais cliniques, avec le potentiel de combler des lacunes des connaissances, notamment concernant l'utilisation de médicaments, dans la pratique clinique de routine.

De plus, ces données du quotidien peuvent permettre d'adapter le traitement aux symptômes du patient, dans une approche de médecine personnalisée. Cette adaptation peut se faire sur la posologie et en particulier sur la fréquence d'administration. En effet, dans la maladie de Parkinson, les symptômes peuvent varier significativement dans le temps, même au cours d'une seule journée. L'adaptation du traitement en fonction des symptômes permettrait une meilleure prise en charge du patient, en particulier si le suivi des symptômes et l'adaptation de la posologie sont réalisés en continu.

L'invention s'inscrit dans ce contexte.

**RÉSUMÉ**

L'invention concerne donc un procédé mis en oeuvre par ordinateur pour déterminer, à un instant courant t, des informations concernant une dose de médicament pour le traitement de la maladie de Parkinson, comprenant :

- recevoir, à l'instant courant t, par une ou plusieurs interfaces d'entrée, des données de biomarqueur relatives à un niveau de biomarqueur représentatif d'un symptôme de la maladie de Parkinson d'un utilisateur, à partir d'un capteur de mesure *in vivo* dudit biomarqueur ;
- recevoir, d'une ou plusieurs mémoires, des données dudit biomarqueur de l'utilisateur antérieures à l'instant courant t;
- recevoir, d'une ou plusieurs mémoires, des informations relatives à des prises d'une dose du médicament pour le traitement de la maladie de Parkinson, par l'utilisateur, antérieures à l'instant courant t;
- déterminer, à l'aide d'un ou plusieurs processeurs, un niveau de biomarqueur de l'utilisateur à partir des données de biomarqueur reçues ;
- déterminer, à l'aide dudit ou desdits processeurs, un niveau d'alerte à partir du niveau de biomarqueur de l'utilisateur et de valeurs limites de référence ;
- déterminer, à l'aide dudit ou desdits processeurs, un taux de changement du niveau de biomarqueur de l'utilisateur à l'aide des données de biomarqueur reçues et de données de biomarqueur précédentes ;
- déterminer, à l'aide dudit ou desdits processeurs, une évolution du niveau de biomarqueur de l'utilisateur à l'aide des données de biomarqueur reçues, des données de biomarqueur précédentes, et de données de référence ;

- déterminer, à l'aide dudit ou desdits processeurs, un niveau de cohérence du niveau de biomarqueur de l'utilisateur par rapport à sa dernière prise d'une dose de médicament pour le traitement de la maladie de Parkinson, à l'aide du niveau du biomarqueur de l'utilisateur, du taux de changement du niveau de biomarqueur de l'utilisateur, de l'évolution du niveau de biomarqueur de l'utilisateur et d'une courbe représentative de l'évolution temporelle d'une concentration théorique dudit médicament dans le corps de l'utilisateur et spécifique à l'utilisateur; et
- déterminer, à l'aide dudit ou desdits processeurs, une dose recommandée de médicament pour le traitement de la maladie de Parkinson au moins sur la base du niveau du biomarqueur de l'utilisateur, du niveau d'alerte et du niveau de cohérence.

Ainsi, le procédé pour déterminer, à un instant courant t, des informations concernant une dose de médicament pour le traitement de la maladie de Parkinson permet, sur la base de mesures courantes de biomarqueur et d'historiques de mesures du même biomarqueur et de prises de doses de médicament d'un utilisateur, de surveiller les symptômes de cet utilisateur et d'adapter en continu la posologie du médicament.

Dans certains modes de réalisation, le procédé comprend en outre : présenter, via une ou plusieurs interfaces de sortie, la dose recommandée de médicament pour le traitement de la maladie de Parkinson à un destinataire.

Avantageusement, le destinateur est l'utilisateur, un personnel de santé, un centre de télésurveillance ou un processeur. Ainsi, l'utilisation du procédé décrit ici est multiple et peut par exemple se rapporter soit à de l'automédication, soit à un suivi adapté de l'utilisateur par le personnel de santé, soit à la surveillance à distance de la réaction de l'utilisateur au médicament, soit à un traitement automatisé de la recommandation déterminée par le procédé.

Dans certains modes de réalisation, le procédé comprend en outre : transmettre, via une ou plusieurs interfaces de sortie, la dose recommandée de médicament pour le traitement de la maladie de Parkinson à un dispositif d'administration de médicament. Ainsi, le procédé décrit ici permet d'automatiser une adaptation de la posologie du médicament, sans intervention humaine.

Dans certains modes de réalisation, les informations relatives à des prises d'une dose du médicament pour le traitement de la maladie de Parkinson comprennent, pour au moins une des prises antérieures à l'instant t, un triplet de données formé d'un type de médicament, d'une valeur de dose et d'un instant de prise.

Dans certains modes de réalisation, le médicament pour le traitement de la maladie de Parkinson comprend de la lévodopa.

Dans certains modes de réalisation :

- le symptôme de la maladie de Parkinson est le tremblement d'une main de l'utilisateur ;
- les données de biomarqueur relatives à ce symptôme reçues sont la fréquence du tremblement et l'amplitude du tremblement ;
- le niveau du biomarqueur est déterminé suite au calcul d'un couple de valeur formé de la fréquence du tremblement et de l'intensité de tremblement normalisée;
- les valeurs limites de référence pour déterminer le niveau d'alerte sont une fréquence de tremblements inférieure ou égale à 3 Hz et une intensité normalisée de tremblement supérieure ou égale à 0,4 ;
- les données de référence pour déterminer l'évolution du niveau de biomarqueur de l'utilisateur se présentent sous la forme d'un diagramme de référence bidimensionnel comprenant un ensemble de points de référence de densité donnée, avec un axe d'abscisses représentant une fréquence de tremblement, et un axe d'ordonnées représentant une intensité normalisée de tremblement, et comprenant également une ligne de référence fixe, nommée ligne de crête.

Avantageusement :

- le diagramme de référence bidimensionnel a été obtenu par une campagne de mesures temporelles de la fréquence et de l'amplitude du tremblement sur un sujet de référence atteint de la maladie de Parkinson pendant un intervalle de temps prédéfini ;
- le diagramme de référence comprend, dans un premier voisinage de la ligne de crête, pour des fréquences supérieures à 7 Hz et des intensités normalisées supérieures à 0,5, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé.

Un autre aspect de l'invention porte sur un dispositif pour déterminer, à un instant courant t, des informations concernant une dose de médicament pour le traitement de la maladie de Parkinson, comprenant :

- une ou plusieurs interfaces d'entrée, configurées pour :

- recevoir, à l'instant courant t, par une ou plusieurs interfaces d'entrée, des données de biomarqueur relatives à un niveau de biomarqueur représentatif d'un symptôme de la maladie de Parkinson d'un utilisateur, à partir d'un capteur de mesure *in vivo* dudit biomarqueur ;
- une ou plusieurs mémoires, configurées pour :

  - stocker des données dudit biomarqueur de l'utilisateur antérieures à l'instant courant t;
  - stocker des informations relatives à des prises d'une dose du médicament pour le traitement de la maladie de Parkinson, par l'utilisateur, antérieures à l'instant courant t;

- un ou plusieurs processeurs, configurés pour :

  - déterminer un niveau de biomarqueur de l'utilisateur à partir des données de biomarqueur reçues ;
  - déterminer un niveau d'alerte à partir du niveau de biomarqueur de l'utilisateur et de valeurs limites de référence ;
  - déterminer un taux de changement du niveau de biomarqueur de l'utilisateur à l'aide des données de biomarqueur reçues et des données de biomarqueur précédentes ;
  - déterminer une évolution du niveau de biomarqueur de l'utilisateur à l'aide des données de biomarqueur reçues, des données de biomarqueur précédentes, et de données de référence ;
  - déterminer un niveau de cohérence du niveau de biomarqueur de l'utilisateur par rapport à sa dernière prise d'une dose de médicament pour le traitement de la maladie de Parkinson , à l'aide du niveau du biomarqueur de l'utilisateur, du taux de changement du niveau de biomarqueur de l'utilisateur, de l'évolution du niveau de biomarqueur de l'utilisateur et d'une courbe représentative de l'évolution temporelle d'une concentration théorique dudit médicament dans le corps de l'utilisateur et spécifique à l'utilisateur ; et
  - déterminer une dose recommandée de médicament pour le traitement de la maladie de Parkinson au moins sur la base du niveau du biomarqueur de l'utilisateur, du niveau d'alerte et du niveau de cohérence.

Avantageusement, le dispositif pour déterminer, à un instant courant t, des informations concernant une dose de médicament pour le traitement de la maladie de Parkinson est configuré pour mettre en oeuvre le procédé pour déterminer, à un instant courant t, des informations concernant une dose de médicament pour le traitement de la maladie de Parkinson comme décrit dans les précédents modes de réalisation.

Dans certains modes de réalisation, le dispositif pour déterminer, à un instant courant t, des informations concernant une dose de médicament pour le traitement de la maladie de Parkinson comprend en outre une interface de sortie configurée pour présenter la dose recommandée de médicament pour le traitement de la maladie de Parkinson.

Un autre aspect de l'invention porte sur un produit programme informatique comportant des instructions pour mettre en oeuvre des étapes du procédé pour déterminer, à un instant courant t, des informations concernant une dose de médicament pour le traitement de la maladie de Parkinson précédemment décrit, lorsque ce programme est exécuté par un processeur.

Encore un autre aspect de l'invention porte sur un support d'enregistrement lisible par ordinateur non-transitoire comprenant les instructions qui lorsqu'elles sont exécutées par un ordinateur, conduisent celui-ci à mettre en oeuvre des étapes du procédé décrit ci-dessus.

**BRÈVE DESCRIPTION DES FIGURES**

**Figure 1** illustre un dispositif pour mettre en oeuvre un procédé pour déterminer, à un instant courant t, des informations concernant une dose de médicament pour le traitement de la maladie de Parkinson selon un ou plusieurs modes de réalisation.

**Figure 2** montre un diagramme bidimensionnel comprenant des données de référence relatives à un symptôme de la maladie de Parkinson qui est le tremblement d'une main.

**Figure 3** représente un ordinogramme d'un exemple d'étapes pour la réalisation d'un procédé pour déterminer, à un instant courant t, des informations concernant une dose de médicament pour le traitement de la maladie de Parkinson.

**Figure 4** représente un exemple de courbe de l'évolution temporelle d'une concentration théorique de médicament dans le corps d'un utilisateur et spécifique à l'utilisateur.

**Figure 5** représente un exemple d'étapes mises en oeuvre pour la réalisation d'une phase de calibration pour obtenir des paramètres spécifiques à un utilisateur, selon un ou plusieurs modes de réalisation.

## DESCRIPTION DÉTAILLÉE

La présente invention concerne un procédé 100 mis en oeuvre par ordinateur pour déterminer, à un instant courant t, des informations concernant une dose de médicament pour le traitement de la maladie de Parkinson, ainsi qu'un dispositif 1 configuré pour mettre en oeuvre le procédé 100.

Comme indiqué précédemment, il peut s'avérer avantageux de tirer parti de données du quotidien de patients atteints de la maladie de Parkinson et prenant un traitement pour cette maladie, pour le suivi des effets de ce traitement et l'extraction d'informations relatives aux doses de médicament(s) prises ou à prendre par les patients. Par exemple, le procédé 100 peut permettre de déterminer une recommandation de dose de médicament à prendre par un patient pour lequel le procédé 100 est mis en oeuvre.

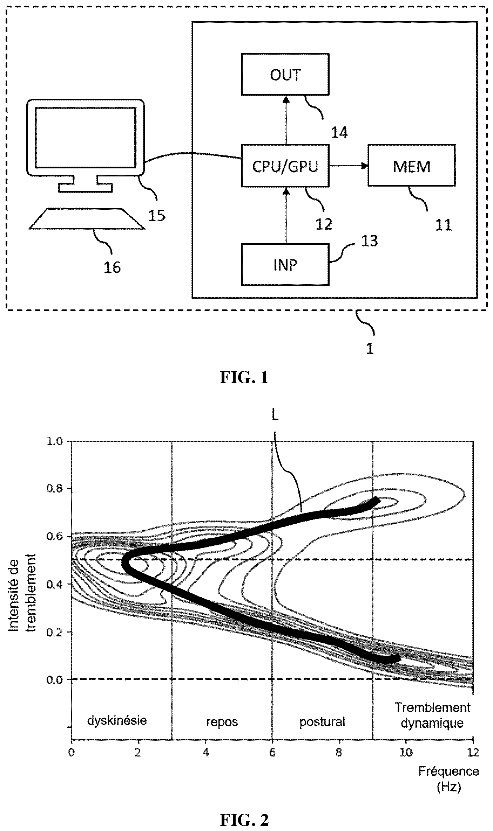

Le procédé 100 peut être mis en oeuvre à l'aide du dispositif 1 tel qu'illustré sur la figure 1, qui est un diagramme schématique montrant les composants d'un exemple du dispositif 1.

Le dispositif 1 peut être mis en oeuvre en tant que dispositif matériel unique, par exemple sous la forme d'un ordinateur personnel (PC) de bureau, d'un ordinateur portable, d'un assistant numérique personnel (PDA), d'un ordiphone, d'une montre connectée, d'un serveur, d'une console ou peut être mis en oeuvre sur des dispositifs matériels interconnectés séparés interconnectés par un ou plusieurs liens de communication, avec des segments câblés et/ou sans fil. Le dispositif 1 peut par exemple être en communication avec un ou plusieurs systèmes informatiques en nuage, un ou plusieurs serveurs ou dispositifs distants pour mettre en oeuvre les fonctions décrites dans le présent document pour le dispositif concerné. Le dispositif 1 peut également être mis en oeuvre lui-même en tant que système informatique en nuage.

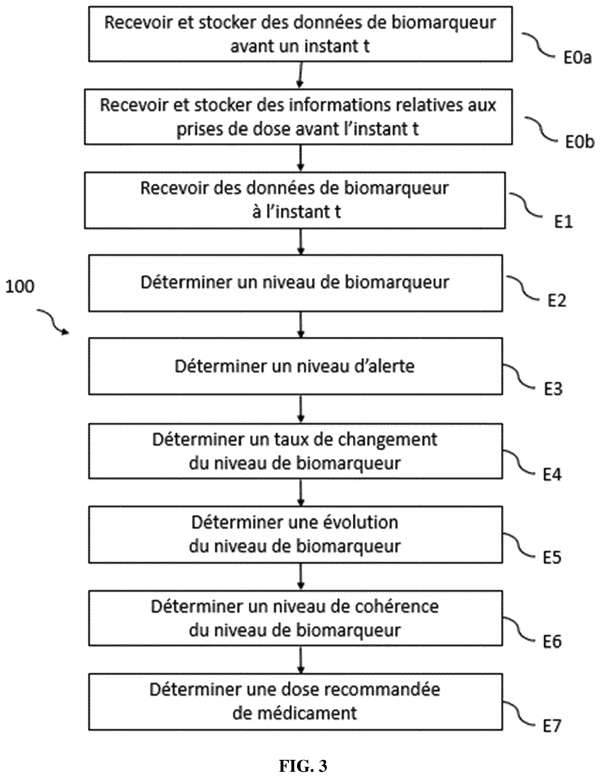

Comme représenté schématiquement sur la figure 1, le dispositif 1 comprend un ordinateur, cet ordinateur comprenant une mémoire 11 pour stocker des instructions de programme chargeables dans un circuit et adaptées pour amener un circuit 12 à exécuter des étapes du procédé de la figure 3, décrite plus loin, lorsque les instructions de programme sont exécutées par le circuit 12. La mémoire 11 peut également stocker des données et des informations utiles pour l'exécution des étapes de la présente invention telles que décrites ci-dessus.

Le circuit 12 peut être par exemple :

- un processeur ou une unité de traitement adapté pour interpréter des instructions dans un langage informatique, le processeur ou l'unité de traitement peut comprendre, être associé ou attaché à une mémoire comprenant les instructions, ou
- l'association d'un processeur/unité de traitement et d'une mémoire, le processeur ou l'unité de traitement étant adapté pour interpréter des instructions dans un langage informatique, la mémoire comprenant lesdites instructions, ou
- une carte électronique dans laquelle les étapes de l'invention sont décrites dans le silicium, ou
- une puce électronique programmable telle qu'une puce FPGA (pour " Field-Programmable Gate Array ").

La mémoire 11 peut comprendre une mémoire vive (RAM), une mémoire cache, une mémoire non volatile, une mémoire de sauvegarde (par exemple, des mémoires programmables ou flash), une mémoire morte (ROM), un disque dur (HDD), un lecteur à état solide (SSD) ou toute combinaison de ceux-ci. La ROM de la mémoire 11 peut être configurée pour stocker, entre autres, un système d'exploitation et/ou un ou plusieurs codes de programmes informatiques d'une ou plusieurs applications logicielles. La RAM de la mémoire 11 peut être utilisée par le circuit 12 pour le stockage temporaire de données.

L'ordinateur peut également comprendre une interface d'entrée 13 pour la réception de données d'entrée et une interface de sortie 14 pour fournir des données de sortie. Des exemples de données d'entrée et de données de sortie seront fournis ultérieurement.

Pour faciliter l'interaction avec l'ordinateur, un écran 15 et un clavier 16 peuvent être fournis et connectés au circuit informatique 12.

Le dispositif 1 est configuré pour mettre en oeuvre le procédé 100 mis en oeuvre par ordinateur pour déterminer, à un instant courant t, des informations concernant une dose de médicament pour le traitement de la maladie de Parkinson.

On suppose que le dispositif 1 reçoit, par son interface d'entrée 13, d'un capteur de mesure *in vivo* d'un biomarqueur, des données relatives à ce biomarqueur. Le biomarqueur est représentatif d'un symptôme de la maladie de Parkinson. Le capteur de mesure est porté par ou en interaction avec un utilisateur atteint de la maladie de Parkinson et prenant un médicament pour le traitement de cette maladie, pour lequel le procédé 100 est mis en oeuvre.

Par « **biomarqueur** », il est entendu une caractéristique définie qui est mesurée comme un indicateur des processus biologiques normaux, des processus pathogènes ou des réactions à une exposition ou une intervention, y compris les interventions thérapeutiques. Typiquement, un biomarqueur est associé à un symptôme d'une pathologie.

5

Par exemple, le symptôme est le tremblement d'une main de l'utilisateur. Des données relatives au biomarqueur représentatif du tremblement sont typiquement acquises au moyen d'une montre connectée portée par l'utilisateur, ou d'un ordiphone porté par la main de l'utilisateur de façon récurrente, la montre connectée ou l'ordiphone comprenant un capteur de type centrale inertielle intégrée ou un accéléromètre.

Dans un autre exemple, le symptôme est le rythme cardiaque de l'utilisateur. Des données relatives au biomarqueur représentatif du rythme cardiaque peuvent être mesurées également au moyen d'une montre connectée portée par l'utilisateur, ou d'un ordiphone porté par la main de l'utilisateur de façon récurrente, la montre connectée ou l'ordiphone comprenant un cardiofréquencemètre et/ou un oxymètre.

Dans un autre exemple, le symptôme est le tremblement de la voix de l'utilisateur. Des données relatives au biomarqueur représentatif à ce tremblement sont par exemple un enregistrement de la voix de l'utilisateur, et peuvent être mesurées au moyen d'un microphone.

Encore dans un autre exemple, le biomarqueur est un biomarqueur de justesse représentatif de la justesse du geste de l'utilisateur lors d'un exercice de calligraphie.

Encore dans un autre exemple, le biomarqueur est un biomarqueur parmi le « finger tapping », le « toe tapping », le « foot taping ».

Le procédé 100 vise à déterminer, à un instant courant t, des informations concernant une dose du ou des médicament(s) pour le traitement de la maladie de Parkinson pris par l'utilisateur. La figure 3 est un exemple d'ordinogramme représentant une suite d'étapes pouvant être réalisées pour implémenter le procédé 100.

Dans une étape E1, le dispositif 1 reçoit, via l'interface d'entrée, des données de biomarqueur (tel qu'illustré précédemment), relatives à un niveau de biomarqueur représentatif d'un symptôme de la maladie de Parkinson de l'utilisateur, à partir d'un capteur de mesure *in vivo* dudit biomarqueur.

Par exemple, lorsque le symptôme est le tremblement d'une main de l'utilisateur, les données reçues du capteur sont des données brutes correspondant aux valeurs d'accélérations selon trois axes, mesurées avec un échantillonnage déterminé, par exemple, toutes les 10 millisecondes. Ces données brutes sont prétraitées afin d'obtenir un couple de valeur formé d'une fréquence du tremblement et de l'amplitude du tremblement.

Dans une étape E0a préalable à l'étape E1, le dispositif 1 a reçu puis stocké dans la mémoire 11 des données du biomarqueur de l'utilisateur antérieures à l'instant courant t.

L'acquisition des données du biomarqueur peut se faire de manière discrète ou en continu. Préférentiellement, les données du biomarqueur de l'utilisateur sont acquises en continu. Alternativement, les données du biomarqueur de l'utilisateur sont acquises de manière discrète. Par exemple, les données du biomarqueur de l'utilisateur sont acquises au moins une fois toutes les 2 heures, de préférence une fois par heure, plus préférentiellement toutes les 30 minutes, plus préférentiellement toutes les 15 minutes. Ces acquisitions fréquentes ou continues sont avantageusement effectuées dans la journée (« journée » étant ici définie comme la période d'activité diurne du sujet, qui dure typiquement 14 à 18h par jour pour un sujet humain).

Par ailleurs, dans une étape E0b préalable à l'étape E1, le dispositif 1 a également reçu puis stocké dans la mémoire 11 des informations relatives à des prises d'une dose de médicament pour le traitement de la maladie de Parkinson, par l'utilisateur, antérieures à l'instant courant t. Par exemple, les informations relatives à des prises d'une dose de médicament pour le traitement de la maladie de Parkinson comprennent, pour au moins une des prises antérieures à l'instant t, un triplet de données formé d'un type de médicament, d'une valeur de dose et d'un instant de prise.

Dans une étape E2, le circuit 12 du dispositif 1 détermine un niveau de biomarqueur de l'utilisateur à partir des données de biomarqueur reçues à l'étape E1.

Par exemple, dans le cas où le symptôme est le tremblement d'une main de l'utilisateur, le niveau de biomarqueur est calculé sur la base du couple de valeurs de fréquence et d'amplitude du tremblement. Pour chaque mesure, l'amplitude est normalisée de sorte à obtenir une valeur d'intensité normalisée de la mesure. La valeur d'intensité normalisée de la mesure est comprise entre 0 et 1. Par exemple, pour normaliser l'amplitude du signal, celle-ci peut être divisée par l'amplitude maximale des tremblements du sujet de référence dont les mesures du biomarqueur ont été utilisées pour créer le diagramme de référence.

Alternativement, toujours dans le cas où le symptôme est le tremblement d'une main de l'utilisateur, la valeur d'intensité normalisée de la mesure peut être obtenue comme suit. Le capteur fournit des données brutes correspondant aux accélérations suivant trois directions perpendiculaires x, y et z. Une analyse en fréquence des données brutes est réalisée à intervalles réguliers, par exemple, toutes les 5 secondes. Par exemple, l'analyse en fréquence peut consister en un calcul de transformée de Fourier, afin d'obtenir le spectre en fréquence des données brutes.

Dans un mode de réalisation, l'intensité de tremblement normalisée qui est utilisée pour le diagramme de référence prédéterminé est calculée sur la base de la puissance crête du spectre en fréquence (« peak power »).

Dans un autre mode de réalisation, l'intensité de tremblement normalisée qui est utilisée pour le diagramme de référence prédéterminé est calculée sur la base d'une puissance fenêtrée (« windowed power » du spectre en fréquence). Par exemple, la puissance fenêtrée peut être calculée par intégration du spectre sur la bande [3, 12 Hz] ou sur la bande [3, 9 Hz].

Dans encore un autre mode de réalisation, l'intensité de tremblement normalisée qui est utilisée pour le diagramme de référence prédéterminé est calculée sur la base de la puissance totale du spectre en fréquence. En pratique, la puissance totale peut être calculée par intégration du spectre sur une bande finie telle que la bande [0, 50 Hz].

Par exemple, l'intensité de tremblement normalisée est une variable composite de la fréquence de tremblement et d'une puissance calculée sur la base du spectre en fréquence, par exemple une puissance crête, une puissance fenêtrée ou une puissance totale. Cette variable composite peut être obtenue par l'implémentation d'un modèle d'apprentissage automatique configuré notamment pour recevoir en entrée le spectre en fréquence complet des tremblements (i.e., aucune bande de fréquence n'est supprimée). Ce mode de réalisation, il est particulièrement avantageux car il permet de compresser l'information concernant toutes les fréquences de tremblement dans une variable composite encodée sans pour autant perdre d'information.

Dans une étape E3, le circuit 12 du dispositif 1 détermine un niveau d'alerte à partir du niveau de biomarqueur et de valeurs limites de référence.

Par « **niveau d'alerte** » il est fait référence à un niveau du biomarqueur au-delà duquel l'état de santé de l'utilisateur est dégradé ou peut se dégrader.

Par exemple, dans le cas où le symptôme est le tremblement d'une main de l'utilisateur, un niveau d'alerte élevé, correspondant à une attention à porter à l'état de l'utilisateur, peut être déterminé si la fréquence de tremblement est inférieure ou égale à 3Hz et si l'intensité normalisée de tremblement est supérieure ou égale à 0,4. Dans un autre exemple, dans le cas où le symptôme est le tremblement d'une main de l'utilisateur, un niveau d'alerte élevé, correspondant à une attention à porter à l'état de l'utilisateur, peut être déterminé si l'intensité normalisée de tremblement a une valeur allant de 0,4 à 0,6, préférentiellement de 0,4 à 0,55.

Dans une étape E4, le circuit 12 du dispositif 1 détermine un taux de changement du niveau de biomarqueur de l'utilisateur à l'aide des données de biomarqueur reçues à l'étape E1 et des données de biomarqueur précédentes obtenues et stockées à l'étape E0a. Par exemple, le taux de changement du niveau du biomarqueur peut correspondre à une vitesse de variation du niveau du biomarqueur à l'instant t par rapport à un instant antérieur. Le « **taux de changement du niveau du biomarqueur** » permet de traduire la vitesse d'évolution de l'état de santé de l'utilisateur, i.e. un changement plus ou moins rapide de son état de santé.

Dans le cas où le symptôme est le tremblement d'une main de l'utilisateur, le taux de changement du niveau du biomarqueur peut correspondre d'une part à la vitesse de variation de la fréquence de tremblement, et d'autre part à la vitesse de variation de l'intensité normalisée du tremblement. Une illustration du taux de changement du niveau de biomarqueur consiste par exemple en la succession d'une amélioration brutale du niveau de biomarqueur, par exemple en environ 5 minutes, suivie d'une dégradation progressive du niveau de biomarqueur.

Dans une étape E5, le circuit 12 du dispositif 1 détermine une évolution du niveau de biomarqueur de l'utilisateur à l'aide des données de biomarqueur reçues à l'étape E1, des données de biomarqueur précédentes obtenues et stockées à l'étape E0a, et de données de référence. L'« **évolution du niveau de biomarqueur »** permet de traduire l'évolution de l'état de santé de l'utilisateur, i.e. une amélioration, une dégradation, ou un maintien de son état de santé.

Dans le cas où le symptôme est le tremblement d'une main de l'utilisateur, les données de référence sont par exemple un diagramme de référence bidimensionnel comprenant un ensemble de points de référence de densité donnée, avec un axe d'abscisses représentant une fréquence de tremblement, et un axe d'ordonnées représentant une intensité normalisée de tremblement, et comprenant également une ligne de référence fixe, nommée ligne de crête.

Par exemple, le diagramme de référence bidimensionnel a été obtenu par une campagne de mesures temporelles de la fréquence et de l'amplitude du tremblement sur un sujet de référence atteint de la maladie de Parkinson pendant un intervalle de temps prédéfini.

La figure 2 montre le diagramme de référence d'un sujet de référence à partir de 86000 mesures prises pendant un intervalle de temps de trois mois. Le diagramme de référence est un diagramme bidimensionnel comprenant un ensemble de points de référence de densité donnée, avec un axe d'abscisses représentant une fréquence de tremblement en Hertz, et un axe d'ordonnées représentant une intensité de tremblement normalisée (i.e., adimensionnelle).

Chaque point de référence du diagramme de référence correspond à l'une des 86000 mesures et présente une densité ou en d'autres termes un nombre d'occurrences pendant l'intervalle de temps de trois mois. L'ensemble des points de référence est réparti en courbes de niveau. Chaque point d'une même courbe de niveau présente la même densité.

Le diagramme de référence prédéterminé est comparable à une photographie longue exposition où sont accumulées des mesures représentatives du biomarqueur pendant l'intervalle de temps de la campagne de mesures, où se sont accumulées les mesures de fréquence et d'intensité de tremblement normalisée.

Trois bandes de fréquence de tremblement sont remarquables sur le diagramme de référence, et repérables au niveau des îlots des courbes de niveau de plus forte densité : autour de 1,9 Hz, bande correspondant à la dyskinésie ; autour de 5 Hz et autour de 9-10 Hz, bande correspondant aux tremblements dynamiques. Ces trois bandes de fréquence correspondent aux fréquences de contraction des muscles caractéristiques de sujets atteints de la maladie de Parkinson.

Il peut être observé que l'ensemble des points de référence sont localisés au voisinage d'une ligne de référence fixe L, dénommée ci-après ligne de crête. Par exemple, la ligne de crête est une ligne d'interpolation passant par les maxima des îlots du diagramme de référence prédéterminé.

Les inventeurs de la présente demande ont en effet découvert que, lorsque des mesures d'un biomarqueur représentatif de tremblements de la main d'un individu atteint de la maladie de Parkinson sont prises, enregistrées sous la forme de points ayant pour coordonnées la fréquence du tremblement et l'intensité normalisée du tremblement, ces points se situaient au voisinage de la ligne de crête du diagramme de référence. Autrement dit, les mesures d'un tel biomarqueur se déplacent globalement autour de la ligne de crête L. La position du point correspondant à la mesure du biomarqueur le long de la ligne de crête L dépend de l'état de santé de l'individu.

Aussi, il est à noter que la zone du diagramme de référence où les intensités normalisées de tremblement sont supérieures à 0,5 correspond à des tremblements perceptibles à l'oeil, tout en étant mesurables par un capteur. Au contraire, la zone du diagramme de référence où les intensités normalisées de tremblement sont inférieures à 0,5 correspond à des tremblements imperceptibles à l'oeil, mais mesurables par un capteur.

Le diagramme de référence comprend, dans un premier voisinage de la ligne de crête, pour des fréquences supérieures à 7 Hz et des intensités normalisées supérieures à 0,5, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé.

Ainsi, dans le cas où le symptôme est le tremblement d'une main de l'utilisateur, il est possible de positionner les données relatives au biomarqueur reçues à l'instant t sur le diagramme bidimensionnel et de déterminer l'évolution du biomarqueur depuis un instant antérieur jusqu'à l'instant t par un trajet sur la ligne de crête. Ce trajet sur la ligne de crête traduit par exemple une hausse de la fréquence de tremblement depuis l'instant antérieur jusqu'à l'instant t, ou une hausse de l'intensité normalisée du tremblement.

Dans une étape E6, le circuit 12 du dispositif 1 détermine un niveau de cohérence du niveau de biomarqueur de l'utilisateur par rapport à sa dernière prise d'une dose de médicament pour le traitement de la maladie de Parkinson, à l'aide du niveau du biomarqueur de l'utilisateur déterminé à l'étape E2, du taux de changement du niveau de biomarqueur de l'utilisateur déterminé à l'étape E4, de l'évolution du niveau de biomarqueur de l'utilisateur déterminé à l'étape E5, et d'une courbe représentative de l'évolution temporelle d'une concentration théorique du médicament dans le corps de l'utilisateur et spécifique à l'utilisateur.

Cette étape E6 vise à comparer l'évolution temporelle, par rapport à une période temporelle passée, du biomarqueur dont les données sont mesurées, à une évolution temporelle d'une concentration théorique du médicament dans le corps de l'utilisateur et spécifique à l'utilisateur, afin d'estimer si l'évolution temporelle du biomarqueur est cohérente, en d'autres termes, en harmonie, avec l'évolution temporelle de la concentration de médicament théorique prédite pour l'utilisateur.

La courbe représentative de l'évolution temporelle d'une concentration théorique du médicament dans le corps de l'utilisateur et spécifique à l'utilisateur peut être obtenue par traitement des données du biomarqueur reçues à l'étape E1, à l'instant courant t, des données du biomarqueur antérieures à l'instant courant t stockées dans la mémoire du dispositif et des informations relatives aux prises de médicament antérieures à l'instant courant t. La courbe représentative peut être obtenue à partir d'une modélisation inspirée de modèles pharmacocinétiques. Un exemple de modélisation va être décrit ci-dessous, dans le cas où le médicament pour le traitement de la maladie de Parkinson est la L-Dopa.

Cet exemple utilise différents paramètres spécifiques au patient $p_n$. Avantageusement, les paramètres spécifiques au patient $p_n$ sont les trois paramètres indépendants suivants :

- $\tau_a$, représentant un facteur apparent de décarboxylase dans le plasma du patient,
- $g_B$, représentant la production endogène de L-dopa dans le cerveau du patient,
- $\Phi_B$ représentant le flux sanguin cérébral.

Les paramètres spécifiques au patient $p_n$ sont obtenus lors d'une phase de calibration utilisant des données de calibration du biomarqueur récoltées pendant une période temporelle $T_{calib}$. La calibration est réalisée sur la base de données collectées pendant une la période temporelle $T_{calib}$.

Ces paramètres sont présents dans les équations suivantes :

$$\tau_a^{-1} = \tau_W^{-1} + \frac{\Phi_B}{V_W}\left(1 - e^{t_B/\tau_a - t_B/\tau_B}\right) \qquad (1)$$

où $\tau_a$ représente un facteur apparent de décarboxylase dans le corps du patient,
rw représente un facteur de décarboxylase dans le volume d'eau du corps du patient,
$\Phi_B$ représente le flux sanguin cérébral, estimé à approximativement 0,88L/minute en journée,
Vw représente le volume d'eau du corps du patient,
$\tau_B$ représente un facteur de décarboxylase dans le cerveau du patient,

$t_B$ représente le temps nécessaire pour qu'un volume $\Phi_B$ dt sanguin cérébral se propage dans le cerveau du patient, estimé à approximativement 75 secondes.

L'équation (1) peut être résolue itérativement.

Le facteur apparent de décarboxylase $\tau_a$ varie entre 75 et 120 minutes d'un patient à l'autre.

Un flux apparent de L-Dopa Ma est définit :

$$M_a = \frac{M + t_B/\tau_B \cdot g_B\left(1 - e^{-t_B/\tau_B}\right)}{1 + \frac{V_B}{V_W} e^{-t_B/\tau_B}} \quad (2)$$

où M représente la variation temporelle de la quantité de L-Dopa ingérée par le patient, en milligrammes par minute, qui est connue,

$g_B$ représente la production endogène de L-Dopa dans le cerveau du patient,

$V_B$ représente le volume d'eau dans le cerveau du patient, estimé à environ 1,1 L,

Vw représente le volume d'eau dans le corps du patient, estimé à environ 40 L.

En particulier, l'expression du paramètre $g_B$ peut être déduite de l'équation (2).

Le facteur apparent de décarboxylase $\tau_a$ varie entre 75 et 120 minutes d'un patient à l'autre.

Pour un jeu de paramètres donné $p_n$, une fonction intermédiaire f(t), ayant pour variable le temps, t, est définie en résolvant df/dt=-f/$\tau_a$,+M. Ainsi, une fonction temporelle tdLED($p_n$,$t_j$) égale à f($t_j$) est définie, où $t_j$ désigne les instants où des données du biomarqueur ont été reçues et stockées jusqu'à l'instant courant t.

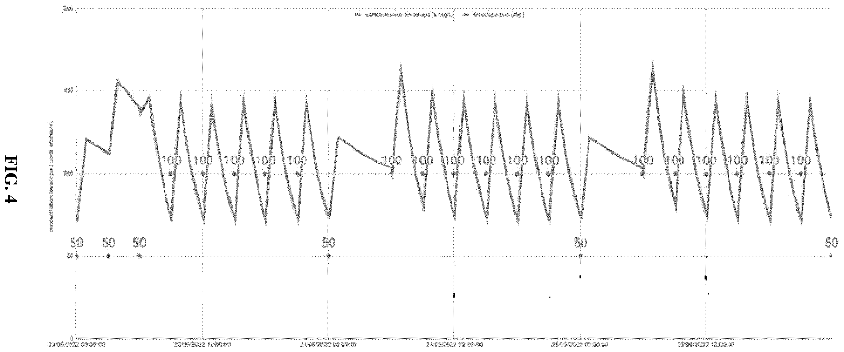

Un exemple de courbe de la fonction tdLED est illustré à la figure 4.

Il est décrit à présent un exemple d'implémentation de la calibration servant à déterminer les paramètres $p_n$ spécifiques à l'utilisateur.

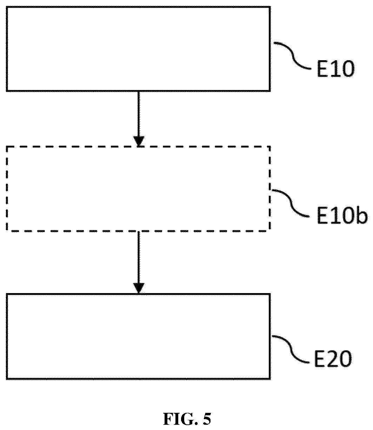

La figure 5 représente un exemple d'étapes mises en oeuvre pour la réalisation de la phase de calibration.

On suppose ici que l'utilisateur possède une prescription décrivant, sur la période temporelle $T_{calib}$, les moments prescrits de prise de médicaments par le patient pendant la période temporelle $T_{calib}$, ainsi que les quantités correspondantes de médicaments. L'un des médicaments correspond à un médicament comprenant de la L-Dopa. Par exemple, les quantités correspondantes sont des concentrations en milligrammes.

Pendant la période temporelle $T_{calib}$, le patient consigne les différents moments effectifs ti de prise de médicaments $M_1$, avec 1 un nombre entier compris entre 1 et L, ainsi que les quantités effectivement prises correspondantes $m_{il}$. Par exemple, la consigne (ti, $m_{il}$) est effectuée sur papier, puis enregistrée informatiquement, par exemple, dans une mémoire d'un dispositif programmable. Ce dispositif programmable peut être le dispositif 1 mais peut être alternativement distinct du dispositif 1. Dans ce cas, les paramètres spécifiques à l'utilisateur (pn) seront envoyés au dispositif 1 préalablement à la mise en oeuvre du procédé 100. Dans un autre exemple, la consigne ($t_i$, $m_{il}$) est effectuée informatiquement dans un fichier numérique stocké dans le dispositif programmable.

En parallèle, pendant la période temporelle $T_{calib}$, des données de calibration vont être enregistrées. Ces mesures sont discrètes ou continues. Les données de calibration sont par exemple de même nature que les données de biomarqueur reçues à l'étape E1 et mesurées alors par le capteur *in vivo*. Ces mesures sont par ailleurs, soit éparses sur la période temporelle $T_{calib}$, soit enregistrées selon un calendrier ordonné sur la période temporelle $T_{calib}$. L'ensemble des mesures enregistrées sur la période temporelle $T_{calib}$ constitue une suite de signaux ($t_j$,$s_j$), avec $t_j$ indiquant un instant de mesure et $s_j$, la mesure du biomarqueur à l'instant $t_j$.

Sur la base de la consigne enregistrée ($t_i$, $m_{il}$) et de la suite de signaux ($t_j$,$s_j$), les paramètres spécifiques au patient vont être déterminés comme suit.

Dans une première étape E10, on réalise une analyse spectrale de la suite de signaux ($t_j$,$s_j$). Par exemple, on calcule la transformée de Fourier de la suite de signaux ($t_j$,$s_j$), afin d'obtenir un spectre correspondant $S_p$. L'étape E10 est effectuée par le dispositif programmable.

Le symptôme associé au biomarqueur présente une signature spectrale caractéristique. Par signature spectrale, on entend le spectre de la mesure temporelle du biomarqueur sur une période donnée, lorsque le patient présente le symptôme correspondant.

Optionnellement, une étape intermédiaire E10b est réalisée suite à la première étape E10. Lors de l'étape intermédiaire E10b, le spectre $S_p$ est identifié à un spectre de référence $S_{ref}$ par apprentissage automatique. Le spectre de référence $S_{ref}$ est caractéristique du symptôme associé au biomarqueur. Par exemple, l'apprentissage automatique est effectué en utilisant une base de données de signatures spectrales de biomarqueurs préalablement enregistrées et collectées. Typiquement, la base de données contient 1500 signatures spectrales correspondant à des sujets atteints par la maladie et 1500 signatures spectrales correspondant à des sujets sains. L'étape E10b peut être effectuée par le dispositif programmable.

Dans une deuxième étape E20, une étape de maximisation de la corrélation de Spearman entre d'une part, le spectres $S_p$ issu de la première étape E10 (ou le spectre $S_{ref}$ issus de l'étape intermédiaire E10b), et d'autre part la fonction tdLED $(p_n, t_j)$ est réalisée. Les valeurs $p_{nopt}$ des paramètres spécifiques à l'utilisateur maximisant la corrélation de Spearman entre la fonction temporelle tdLED $(p_n,t_j)$ et les spectres $S_p$ (ou $S_{ref}$) sont celles utilisées par la suite pour les paramètres spécifiques. La deuxième étape E20 peut être effectuée par le dispositif programmable.

Par exemple, un algorithme de Monte Carlo peut être utilisé pour déterminer les valeurs $p_{nopt}$. Alternativement, une technique de gradient conjugué peut être utilisé. De manière générale, toute technique d'optimisation connue de l'homme du métier peut être utilisée.

Dans le cas où le médicament comprend de la L-dopa, lors de l'étape E6, le niveau de biomarqueur et son taux de changement, et l'évolution du biomarqueur déterminée à l'aide des données de référence, sont utilisés en combinaison avec la courbe tdLED pour déterminer un niveau de cohérence par rapport à la dernière prise de médicament. Le niveau de cohérence est élevé si le niveau de biomarqueur, son taux et changement et son évolution sont cohérent avec la concentration théorique du médicament dans le corps de l'utilisateur.

Dans une étape E7, le circuit 12 du dispositif 1 détermine une dose recommandée de médicament au moins sur la base du niveau du biomarqueur de l'utilisateur, du niveau d'alerte et du niveau de cohérence.

La dose recommandée peut être nulle, dans le cas où il est déterminé qu'il n'est pas recommandé de prendre une nouvelle dose de médicament.

Selon un mode de réalisation, le médicament pour le traitement de la maladie de Parkinson comprend de la lévodopa (L-dopa). Le médicament peut être une composition pharmaceutique à libération immédiate ou prolongée. Dans le cas d'un médicament à libération prolongée, la lévodopa peut être administrée sous forme combinée avec une ou plusieurs substances, telles que le bensérazide, la carbidopa ou l'entacapone.

Afin de déterminer une dose recommandée de médicament, le dispositif 1 comprend en outre des données de posologie de référence, incluant par exemple, pour chaque médicament pouvant être utilisé pour le traitement de la maladie de Parkinson, des valeurs de doses unitaires usuelles, des intervalles d'administration minimums entre deux prises, des doses maximum journalières.

Par exemple, lorsque le médicament comprend de la L-dopa, et qu'il est recommandé de prendre une dose de médicament, la dose recommandée est avantageusement une dose allant de 50 mg à 250 mg de L-dopa par prise. L'intervalle entre deux doses est de préférence d'au moins 1 heure. Le nombre de doses administrées est de préférence d'au maximum 12 doses par jour (de préférence 10 doses par jour, plus préférentiellement 8 doses par jour) et/ou au maximum de 1200 mg de L-dopa par jour.

Avantageusement, le procédé de l'invention permet de déterminer le moment le plus opportun pour administrer une dose de médicament pour le traitement de la maladie de Parkinson. Le procédé de l'invention permet notamment de recommander l'administration d'une nouvelle dose de médicament avant que la dose précédente ne fasse plus complètement d'effet, afin d'éviter une dégradation trop importante de l'état du patient entre deux doses, mais pas trop précocement pour éviter un surdosage et ses effets indésirables associés. Ainsi, l'efficacité du médicament est augmentée et la qualité de vie du patient est améliorée.

Par exemple, dans le cas où le symptôme est le tremblement de la main et qu'une dose de médicament a été prise très récemment, se traduisant par une courbe tdLED montrant une concentration théorique de L-dopa élevée ou en augmentation :

- si le trajet représentant l'évolution du biomarqueur du tremblement sur le diagramme bidimensionnel passe de la première zone (i.e. correspondant à un état de santé dégradé) à une deuxième zone (i.e. correspondant à un état de santé moins dégradé), et que le taux de changement du niveau de biomarqueur est élevé, avec une diminution de la fréquence et/ou de l'amplitude des tremblements, alors le niveau de cohérence est élevé : la L-dopa a commencé à faire effet et il existe encore dans le corps de l'utilisateur une quantité de L-dopa non encore métabolisée qui est encore disponible pour continuer à faire effet et agir sur les symptômes. Dans ce cas, il n'est pas nécessaire d'envisager la prise d'une nouvelle dose de médicament, celle venant d'être prise devrait être suffisante pour améliorer ou maintenir l'état de santé de l'utilisateur ;

- de même, si le trajet représentant l'évolution du biomarqueur du tremblement sur le diagramme bidimensionnel reste situé dans la première zone (i.e. correspondant à un état de santé dégradé), mais que le taux de changement du niveau de biomarqueur est élevé, avec une diminution de la fréquence et/ou de l'amplitude des tremblements, alors le niveau de cohérence est également élevé : la L-dopa commence à faire effet et il existe dans le corps de l'utilisateur une quantité de L-dopa non encore métabolisée qui est disponible pour faire effet et diminuer la fréquence et/ou l'intensité des tremblements et donc aller vers un état de santé moins dégradé. Dans ce cas, il n'est pas non plus nécessaire d'envisager la prise d'une nouvelle dose de médicament, celle venant d'être prise devrait être suffisante pour continuer d'améliorer l'état de santé de l'utilisateur ;

- de même, si le trajet représentant l'évolution du biomarqueur du tremblement sur le diagramme bidimensionnel reste situé dans la deuxième zone (i.e. correspondant à un état de santé moins dégradé), et que le taux de changement du niveau de biomarqueur est faible, alors le niveau de cohérence est également élevé : la L-dopa continue à faire effet et il reste dans le corps de l'utilisateur une quantité de L-dopa non encore métabolisée qui est disponible pour faire effet et limiter la fréquence et/ou l'intensité des tremblements et donc rester dans un état de santé moins dégradé. Dans ce cas, il n'est pas non plus nécessaire d'envisager la prise d'une nouvelle dose de médicament, celle venant d'être prise devrait être suffisante pour continuer de maintenir l'état de santé de l'utilisateur ;

- au contraire, si le trajet représentant l'évolution du biomarqueur du tremblement sur le diagramme bidimensionnel reste situé dans la première zone (i.e. correspondant à un état de santé dégradé), et que le taux de changement du niveau de biomarqueur est faible, alors le niveau de cohérence est faible : la L-dopa présente aurait dû faire effet alors que cela ne se traduit pas sur le biomarqueur. Dans ce cas, il faut envisager la prise d'une nouvelle dose de médicament, celle venant d'être prise n'étant a priori pas suffisante pour améliorer l'état de santé de l'utilisateur.

Dans un autre exemple, dans le cas où le symptôme est le tremblement de la main et qu'une dose de médicament n'a été prise depuis un moment, se traduisant par une courbe tdLED montrant une concentration théorique de L-dopa faible ou en diminution :

- si le trajet représentant l'évolution du biomarqueur du tremblement sur le diagramme bidimensionnel passe d'une deuxième zone (i.e. correspondant à un état de santé moins dégradé) à la première zone (i.e. correspondant à un état de santé dégradé), et que le taux de changement du niveau de biomarqueur est élevé avec une augmentation de la fréquence et/ou de l'amplitude des tremblements, alors le niveau de cohérence est élevé : il n'y a plus assez de L-dopa non encore métabolisée dans le corps du patient et elle ne fait plus assez d'effet sur les tremblements qui s'intensifient. Dans ce cas, il est nécessaire d'envisager la prise d'une nouvelle dose de médicament, la dose précédente n'étant plus suffisante pour maintenir l'état de santé de l'utilisateur ;

- de même, si le trajet représentant l'évolution du biomarqueur du tremblement sur le diagramme bidimensionnel est situé dans la deuxième zone (i.e. correspondant à un état de santé moins dégradé), et que le taux de changement du niveau de biomarqueur est élevé avec une augmentation de la fréquence et/ou de l'amplitude des tremblements, alors le niveau de cohérence est également élevé : là encore, il n'y a plus assez de L-dopa disponible pour agir sur les symptômes qui s'amplifient. Dans ce cas, il est également nécessaire d'envisager la prise d'une nouvelle dose de médicament, la dose précédente n'étant plus suffisante pour maintenir l'état de santé de l'utilisateur ;

- au contraire, si le trajet représentant l'évolution du biomarqueur du tremblement sur le diagramme bidimensionnel reste situé dans la deuxième zone (i.e. correspondant à un état de santé moins dégradé) et que le taux de changement du niveau de biomarqueur est faible, alors le niveau de cohérence est faible : la faible concentration théorique en L-dopa présente dans le corps de l'utilisateur semble encore faire effet sur les symptômes alors qu'on pouvait s'attendre à ce qu'elle ne soit plus suffisante. Dans ce cas, on peut envisager de retarder la prise d'une nouvelle dose de médicament par rapport à la fréquence prescrite.

Avantageusement, le procédé 100 comprend une étape de présentation, via l'interface de sortie 14 du dispositif 1, de la recommandation de dose de médicament pour le traitement de la maladie de Parkinson à un destinataire.

Avantageusement, le destinataire peut être soit l'utilisateur lui-même, soit un personnel de santé, soit un centre de télésurveillance, ou un autre processeur. Autrement dit, le procédé 100 permet diverses utilisations de la recommandation de dose de médicament déterminée.

Par exemple, le procédé 100 permet, lorsque le destinataire est l'utilisateur lui-même, à ce dernier de contrôler de manière autonome, de manière suivie, ses prises de doses de médicament, et par exemple de rectifier un oubli de prise de dose de médicament.

Dans un autre exemple, le procédé 100 permet, lorsque le destinataire est un personnel de santé, à ce dernier d'adapter la posologie du médicament, à partir de l'analyse de l'évolution du niveau de biomarqueur déterminée notamment à l'aide des données du biomarqueur de l'utilisateur antérieures à l'instant courant t et du niveau de cohérence déterminé.

Encore dans un autre exemple, le procédé 100 permet, lorsque le destinataire est un centre de surveillance, de contrôler à distance la réaction de l'utilisateur à la posologie actuelle et d'ajuster, au besoin, cette dernière.

Encore dans un autre exemple, lorsque le destinataire est un autre processeur, le procédé 100 permet un traitement automatisé de la recommandation de dose de médicament déterminée.

Dans certains modes de réalisation, le procédé 100 comprend une étape de transmission, via l'interface de sortie 14 du dispositif 1, de la recommandation de dose de médicament pour le traitement de la maladie de Parkinson à un dispositif d'administration de médicament, tel qu'un système de perfusion entérale. Ceci permet d'adapter de manière automatisée la posologie du médicament pour le traitement de la maladie de Parkinson.

Ainsi, l'un des avantages du procédé 100 est de permettre de suivre l'évolution des symptômes d'un utilisateur atteint de la maladie de Parkinson et d'adapter la posologie du médicament que ce dernier prend pour le traitement de cette maladie.

**Revendications**

1. Procédé mis en oeuvre par ordinateur pour déterminer, à un instant courant t, des informations concernant une dose de médicament pour le traitement de la maladie de Parkinson, comprenant :

    recevoir, à l'instant courant t, par une ou plusieurs interfaces d'entrée, des données de biomarqueur relatives à un niveau de biomarqueur représentatif d'un symptôme de la maladie de Parkinson d'un utilisateur, à partir d'un capteur de mesure *in vivo* dudit biomarqueur ;
    recevoir, d'une ou plusieurs mémoires, des données dudit biomarqueur de l'utilisateur antérieures à l'instant courant t;
    recevoir, d'une ou plusieurs mémoires, des informations relatives à des prises d'une dose du médicament pour le traitement de la maladie de Parkinson, par l'utilisateur, antérieures à l'instant courant t;
    déterminer, à l'aide d'un ou plusieurs processeurs, un niveau de biomarqueur de l'utilisateur à partir des données de biomarqueur reçues ;
    déterminer, à l'aide dudit ou desdits processeurs, un niveau d'alerte à partir du niveau de biomarqueur de l'utilisateur et de valeurs limites de référence ;
    déterminer, à l'aide dudit ou desdits processeurs, un taux de changement du niveau de biomarqueur de l'utilisateur à l'aide des données de biomarqueur reçues et de données de biomarqueur précédentes ;
    déterminer, à l'aide dudit ou desdits processeurs, une évolution du niveau de biomarqueur de l'utilisateur à l'aide des données de biomarqueur reçues, des données de biomarqueur précédentes, et de données de référence ;
    déterminer, à l'aide dudit ou desdits processeurs, un niveau de cohérence du niveau de biomarqueur de l'utilisateur par rapport à sa dernière prise d'une dose de médicament pour le traitement de la maladie de Parkinson, à l'aide du niveau du biomarqueur de l'utilisateur, du taux de changement du niveau de biomarqueur de l'utilisateur, de l'évolution du niveau de biomarqueur de l'utilisateur et d'une courbe représentative de l'évolution temporelle d'une concentration théorique dudit médicament dans le corps de l'utilisateur et spécifique à l'utilisateur; et
    déterminer, à l'aide dudit ou desdits processeurs, une dose recommandée de médicament pour le traitement de la maladie de Parkinson au moins sur la base du niveau du biomarqueur de l'utilisateur, du niveau d'alerte et du niveau de cohérence.

2. Le procédé selon la revendication 1, comprenant en outre :
    présenter, via une ou plusieurs interfaces de sortie, la dose recommandée de médicament pour le traitement de la maladie de Parkinson à un destinataire.

3. Le procédé selon la revendication 2, dans lequel le destinataire est l'utilisateur, un personnel de santé, un centre de télésurveillance ou un processeur.

4. Le procédé selon l'une quelconque des revendications 1 à 3, comprenant en outre :
    transmettre, via une ou plusieurs interfaces de sortie, la dose recommandée de médicament pour le traitement de la maladie de Parkinson à un dispositif d'administration de médicament.

5. Le procédé selon l'une quelconque des revendications 1 à 4, dans lequel lesdites informations relatives à des prises d'une dose du médicament pour le traitement de la maladie de Parkinson comprennent, pour au moins une des prises antérieures à l'instant t, un triplet de données formé d'un type de médicament, d'une valeur de dose et d'un instant de prise.

6. Le procédé selon l'une quelconque des revendications **1** à **5**, dans lequel le médicament pour le traitement de la maladie de Parkinson comprend de la lévodopa.

7. Le procédé selon l'une quelconque des revendications **1** à **6**, dans lequel :

    le symptôme de la maladie de Parkinson est le tremblement d'une main de l'utilisateur ;
    les données de biomarqueur relatives à ce symptôme reçues sont la fréquence du tremblement et l'amplitude du tremblement ;
    le niveau du biomarqueur est déterminé suite au calcul d'un couple de valeur formé de la fréquence du tremblement et de l'intensité de tremblement normalisée ;

les valeurs limites de référence pour déterminer le niveau d'alerte sont une fréquence de tremblements inférieure ou égale à 3 Hz et une intensité normalisée de tremblement supérieure ou égale à 0,4 ;

les données de référence pour déterminer l'évolution du niveau de biomarqueur de l'utilisateur se présentent sous la forme d'un diagramme de référence bidimensionnel comprenant un ensemble de points de référence de densité donnée, avec un axe d'abscisses représentant une fréquence de tremblement, et un axe d'ordonnées représentant une intensité normalisée de tremblement, et comprenant également une ligne de référence fixe, nommée ligne de crête.

8. Le procédé selon la revendication **7**, dans lequel :

- le diagramme de référence bidimensionnel a été obtenu par une campagne de mesures temporelles de la fréquence et de l'amplitude du tremblement sur un sujet de référence atteint de la maladie de Parkinson pendant un intervalle de temps prédéfini ;

- le diagramme de référence comprend, dans un premier voisinage de la ligne de crête, pour des fréquences supérieures à 7 Hz et des intensités normalisées supérieures à 0,5, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé.

9. Dispositif pour déterminer, à un instant courant t, des informations concernant une dose de médicament pour le traitement de la maladie de Parkinson, comprenant :

- une ou plusieurs interfaces d'entrée, configurées pour :

- recevoir, à l'instant courant t, par une ou plusieurs interfaces d'entrée, des données de biomarqueur relatives à un niveau de biomarqueur représentatif d'un symptôme de la maladie de Parkinson d'un utilisateur, à partir d'un capteur de mesure *in vivo* dudit biomarqueur ;

- une ou plusieurs mémoires, configurées pour :

- stocker des données dudit biomarqueur de l'utilisateur antérieures à l'instant courant t;
- stocker des informations relatives à des prises d'une dose du médicament pour le traitement de la maladie de Parkinson, par l'utilisateur, antérieures à l'instant courant t;

- un ou plusieurs processeurs, configurés pour :

- déterminer un niveau de biomarqueur de l'utilisateur à partir des données de biomarqueur reçues ;
- déterminer un niveau d'alerte à partir du niveau de biomarqueur de l'utilisateur et de valeurs limites de référence ;
- déterminer un taux de changement du niveau de biomarqueur de l'utilisateur à l'aide des données de biomarqueur reçues et des données de biomarqueur précédentes ;
- déterminer une évolution du niveau de biomarqueur de l'utilisateur à l'aide des données de biomarqueur reçues, des données de biomarqueur précédentes, et de données de référence ;
- déterminer un niveau de cohérence du niveau de biomarqueur de l'utilisateur par rapport à sa dernière prise d'une dose de médicament pour le traitement de la maladie de Parkinson , à l'aide du niveau du biomarqueur de l'utilisateur, du taux de changement du niveau de biomarqueur de l'utilisateur, de l'évolution du niveau de biomarqueur de l'utilisateur et d'une courbe représentative de l'évolution temporelle d'une concentration théorique dudit médicament dans le corps de l'utilisateur et spécifique à l'utilisateur ; et
- déterminer une dose recommandée de médicament pour le traitement de la maladie de Parkinson au moins sur la base du niveau du biomarqueur de l'utilisateur, du niveau d'alerte et du niveau de cohérence.

10. Dispositif selon la revendication **9**, configuré pour mettre en oeuvre un procédé selon l'une des revendications **1** à **8**.

11. Dispositif selon l'une des revendications **9** ou **10**, comprenant en outre une interface de sortie configurée pour présenter la dose recommandée de médicament pour le traitement de la maladie de Parkinson.

12. Produit programme informatique comportant des instructions pour mettre en oeuvre des étapes du procédé selon l'une des revendications **1** à **8**, lorsque ce programme est exécuté par un processeur.

**FIG. 1**

**FIG. 2**

Recevoir et stocker des données de biomarqueur avant un instant t — E0a

Recevoir et stocker des informations relatives aux prises de dose avant l'instant t — E0b

Recevoir des données de biomarqueur à l'instant t — E1

100

Déterminer un niveau de biomarqueur — E2

Déterminer un niveau d'alerte — E3

Déterminer un taux de changement du niveau de biomarqueur — E4

Déterminer une évolution du niveau de biomarqueur — E5

Déterminer un niveau de cohérence du niveau de biomarqueur — E6

Déterminer une dose recommandée de médicament — E7

**FIG. 3**

**FIG. 4**

FIG. 5

Europäisches
Patentamt

European
Patent Office

Office européen
des brevets

**RAPPORT DE RECHERCHE EUROPEENNE**

Numéro de la demande

**EP 23 30 6220**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | BAUCUM MATT ET AL: "Optimizing Patient-Specific Medication Regimen Policies Using Wearable Sensors in Parkinson's Disease", MANAGEMENT SCIENCE, 10 avril 2023 (2023-04-10), XP093101558, DOI: https://doi.org/10.1287/mnsc.2023.4747 Extrait de l'Internet: URL:https://www.researchgate.net/profile/Matthew-Baucum/publication/351391352_Optimizing_Patient-Specific_Medication_Regimen_Policies_Using_Wearable_Sensors_in_Parkinson's_Disease/links/636031b08d4484154a4f35de/Optimizing-Patient-Specific-Medication-Regimen-Policies-Using-Wearable-Sensors-in-Parkinsons-> | 1-6,9-12 | INV. A61B5/11 A61B5/00 |
| A | * page 1 – page 12 *<br>_____ | 7,8 | |
| A | US 11 367 519 B1 (HELDMAN DUSTIN A [US] ET AL) 21 juin 2022 (2022-06-21) * colonne 10 – colonne 36; revendications 1-2; figures 1-11 *<br>_____ | 1-12 | DOMAINES TECHNIQUES RECHERCHES (IPC)<br><br>A61B |
| A | ROBERT I GRIFFITHS ET AL: "Automated Assessment of Bradykinesia and Dyskinesia in Parkinson's Disease", JOURNAL OF PARKINSON'S DISEASE, vol. 2, no. 1, 1 janvier 2012 (2012-01-01), pages 47-55, XP055300735, DOI: 10.3233/JPD-2012-11071 * Measurment of bradykinesia and dyskinesia by algorithm; page 48 – page 49 *<br>_____ | 1-12 | |

Le présent rapport a été établi pour toutes les revendications

| Lieu de la recherche | Date d'achèvement de la recherche | Examinateur |
|---|---|---|
| La Haye | 15 novembre 2023 | Hirsch, Arthur |

# EP 4 491 112 A1

## ANNEXE AU RAPPORT DE RECHERCHE EUROPEENNE
## RELATIF A LA DEMANDE DE BREVET EUROPEEN NO.

**EP 23 30 6220**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**15-11-2023**

| Document brevet cité au rapport de recherche | Date de publication | Membre(s) de la famille de brevet(s) | Date de publication |
|---|---|---|---|
| US 11367519 B1 | 21-06-2022 | AUCUN | |

Pour tout renseignement concernant cette annexe : voir Journal Officiel de l'Office européen des brevets, No.12/82